# EUROPEAN PATENT SPECIFICATION

(11) **EP 1 214 399 B1**
(45) Date of publication and mention of the grant of the patent: **11.07.2007**
(21) Application number: 00960038.8
(22) Date of filing: 08.09.2000
(51) Int. Cl.: C12N 9/12, C07K 14/555

(54) **HIGH LEVEL CYTOKINE PRODUCTION WITH ENHANCED CELL VIABILITY**
HOHE ZYTOKINPRODUKTION MIT VERBESSERTE ZELLLEBENSFÄHIGKEIT
PRODUCTION DE CYTOKINE DE HAUT NIVEAU A VIABILITE CELLULAIRE RENFORCEE

(30) Priority: 08.09.1999 US 152854 P
(43) Date of publication of application: 19.06.2002
(73) Proprietor: Genetrol Biotherapeutics, Oakland, CA 94606 (US)
(72) Inventor: LAU, Allan, S., 62 Mt. Davis Road, Pok Fu Lam (HK); BROWNING, Laura, Brentwood, CA 94513 (US); KIEFER, Michael, C., Clayton, CA 94517 (US)
(74) Representative: Vossius & Partner
(86) International application number: PCT/US2000/024657
(87) International publication number: WO 2001/018185

(56) References cited:
- WO-A-97/08292
- WO-A-97/08324
- WO-A-98/00013
- BALACHANDRAN SIDDHARTH ET AL: "Activation of the dsRNA-dependent protein kinase, PKR, induces apoptosis through FADD-mediated death signaling." EMBO (EUROPEAN MOLECULAR BIOLOGY ORGANIZATION) JOURNAL, vol. 17, no. 23, 1 December 1998 (1998-12-01), pages 6888-6902, XP002159409 ISSN: 0261-4189
- GIL JESUS ET AL: "Induction of apoptosis by double-stranded-RNA-dependent protein kinase (PKR) involves the alpha subunit of eukaryotic translation initiation factor 2 and NF-kappaB." MOLECULAR AND CELLULAR BIOLOGY, vol. 19, no. 7, July 1999 (1999-07), pages 4653-4663, XP002159410 ISSN: 0270-7306
- IMAIZUMI KAZUNORI ET AL: "The cell death-promoting gene DP5, which interacts with the BCL2 family, is induced during neuronal apoptosis following exposure to amyloid beta protein" JOURNAL OF BIOLOGICAL CHEMISTRY,AMERICAN SOCIETY OF BIOLOGICAL CHEMISTS, BALTIMORE, MD,US, vol. 274, no. 12, 19 March 1999 (1999-03-19), pages 7975-7981, XP002156341 ISSN: 0021-9258
- HAN D K M ET AL: "MRIT, A NOVEL DEATH-EFFECTOR DOMAIN-CONTAINING PROTEIN, INTERACTS WITH CASPASES AND BCLXL AND INITIATES CELL DEATH" PROCEEDINGS OF THE NATIONAL ACADEMY OF SCIENCES OF USA,US,NATIONAL ACADEMY OF SCIENCE. WASHINGTON, vol. 94, no. 21, 14 October 1997 (1997-10-14), pages 11333-11338, XP002071904 ISSN: 0027-8424
- GARLAND JOHN M ET AL: "Energy metabolism during apoptosis: bcl-2 promotes survival in hematopoietic cells induced to apoptose by growth factor withdrawal by stabilizing a form of metabolic arrest." JOURNAL OF BIOLOGICAL CHEMISTRY, vol. 272, no. 8, 1997, pages 4680-4688, XP002159411 ISSN: 0021-9258
- CHITTENDEN T ET AL: "INDUCTION OF APOPTOSIS BY THE BCL-2 HOMOLOGUE BAK" NATURE,GB,MACMILLAN JOURNALS LTD. LONDON, vol. 374, 20 April 1995 (1995-04-20), pages 733-736, XP002910967 ISSN: 0028-0836

## Description

The present invention relates to methods for enhancing the production of cytokines in cell culture by inhibiting apoptosis associated with cytokine synthesis, particularly under conditions of PKR overproduction.

### References

Abbas, AK, et al., Eds., CELLULAR AND MOLECULAR IMMUNOLOGY, 3rd edition, WB Saunders Co., 256-257, 1997).
Antonelli, G., J Interferon Cytokine Res (17)Suppl 1:S39-S46, (1997).
Antonelli, et al., J. Inf. Disease 163:882-885 (1991).
Ausubel, FM, et al., in CURRENT PROTOCOLS IN MOLECULAR BIOLOGY, John Wiley and Sons, Inc., Media, PA (1992).
Baker, S.J., et al., Science 249(4971):912-5, (1990).
Balachandran, S., et al., EMBO J. 17:6888-6902, (1998).
Balkwill FR and Burke F, Immunology Today, 10(9):299, (1989).
Boise, Thompson. Current Topics Microbiol Immunol 200:107-121, (1995).
Chinnaiyen, D., et al., Cell 81:505-512, (1995).
Chong KL et al., EMBO J. 11(4):1553-62 (1992).
Clemens MJ and Bommer UA, Int J Biochem Cell Biol, 31(1):1-23, (1999).
Clemens MJ and Elia A, J Interferon Cytokine Res, 17(9):503-24, (1997).
Clark S and Kamen R, Science, 236:1229-1237, (1987).
Cohen, J.J., Immunol Today 14(3):126-130, (1993).
Cohen, J.J., et al., Annu Rev Immunol 10:267-293, (1992).
Der, D., and Lau, A.S., Proc Natl Acad Sci USA, 92:8841-8845, (1995).
Deutscher, METHODS IN ENZYMOLOGY 182, 1990; Scopes, PROTEIN PURIFICATION: PRINCIPLES AND PRACTICE, Springer-Verlag, New York (1982).
Donze, O., et al., Virol 256:322-329, (1999).
Dressler, K.A., et al., Science 255:1715-1718, (1992).
Feng GS et al, Proc Natl Acad Sci U S A 89(12):5447-51 (1992).
Galabru, J., and Hovanessian, A., J. Biol. Chem. 262:15538-15544 (1987).
Garcia, I., et al., Science 258:302-304, (1992).
Guy, G.R., et al., J Biol Chem 267(3):1846-1852, (1992).
Harlow and Lane, ANTIBODIES: A LABORATORY MANUAL, Cold Spring Harbor Pubs., N.Y. (1988).
Heller, R.A., et al., Cell 70(1):47-56, (1992).
Hershey, J.W.B., Ann. Rev. Biochem. 60:717-755, (1991).
Hopp et al., Biotechnology 6: 1204-1210, 1988.
Huang et al., Oncogene 14: 405-414,1997.
Kane, D.J., et al., Science 262:1274-1277, (1993).
Korsmeyer, S.J., Blood 80(4):879-886, (1992).
Koromilas et al., Science 257:1685, 1992.
Kosik, K.S., Science 256:780-783, (1992).
Kumar, A., et al Proc Natl Acad Sci USA 91:6288-6292, (1994).
Larrick, J.W., and Wright, S.C., FASEB J 4:215-3223, (1990).
Lau, A.S., et al., Pediat Infect Dis J, CME Review 15:563-575, (1996).
Levine, A.J., Annu Rev Biochem 62:623-651, (1993).
Liddil, J.D., et al., Cancer Res 49:2722-2728, (1989).
Meurs EF et al., J Virol. 66(10):5804-14 (1992).
Meurs, E., and Hovanessian, A.G., Cell 62:379-390, (1990).
Nagata, S., and Suda, T., Immunol Today 16(1):39-43, (1995).
Obeid, L.M., et al., Science 259:1769-1771, (1993).
Oehm, A., et al., J Biol Chem 267(15):10709-10715, (1992).
Orrenius, S., J Intern Med 237:529-536, (1995).
Reed, J., et al., Nature 336:259-261, (1988).
Reed, J., et al., Exp Cell Research 195:277-283, (1991).
Rubin, B.Y., et al., Cancer Res 48:6006-6010, (1988).
Sambrook J, et al., in MOLECULAR CLONING: A LABORATORY MANUAL, Cold Spring Harbor Laboratory Press, Cold Spring Harbor, NY, (1989).
Schendel, Cell Death Differentiation 5(5):372-380, (1998).
Schulze-Osthoff, K., et al., Eur J Biochem 254:439-459, (1998).
Sen, G.C., and Lengyel, P., J Biol Chem 267:5017-5020, (1992).
Stellar, H., Science 267:1445-1449, (1995).
Srivastave, S., et al., J Biol Chem 273:2416-2423, (1998).
Taylor, J.L., and Grossberg, S.E., Virus Research 15:126, (1990).
Thompson, C.B., Science 267:1456-1462, (1995).
Tracey, K.J., and Cerami, A., Annu Rev Cell Biol. 9:317-343, (1993).
Van Lint, J., et al., J Biol Chem 267(36):25916-25921, (1992).
Vaux, D.L., Proc Natl Acad Sci USA 90:786-789, (1993).
Williams, B.R.G., Eur J Biochem. 200:111, (1991).
Williams, B.R.G., Seminars in Oncology 24(S9):70-77, (1997).
Wong G and Clark S, Immunology Today, 9(5):137, 1988.
Yeh, et al., Science 279:1954-1958, (1998).
Yeung, M.C., et al., Proc Natl Acad Sci USA 93:12451-12455, (1996).
Yeung, M., and Lau, AS., J Biol Chem 273:25198-25202, (1998).
Yeung, M., et al., AIDS 12:349-354, (1998).
Yonehara, S., et al., J Exp Med 169(5):1747-1756, (1989).
Zamanian-Daryoush M, et al., Oncogene 14;18(2):315-26, (1999).

Infection by pathogens including viruses, bacteria, and parasites results in activation of the host immune system and signaling by various molecules, such as cytokines, resulting in mobilization of multiple branches of the immune system. Cytokines are a rapidly growing collection of potent, pleiotropic polypeptides that act as local and/or systemic intercellular regulatory factors. (See, *for example,* Balkwill and Burke, 1989; Wong and Clark, 1988; and Clark and Kamen, 1987.) They play crucial roles in many biologic processes, such as immunity, inflammation, and hematopoiesis, and are produced by diverse cell types including fibroblasts, endothelial cells, macrophages/monocytes, and lymphocytes. To date, a large number of cytokines have been identified, including interferons (IFNs), tumor necrosis factors (TNFs), interleukins (ILs), growth factors (*for example,* epidermal growth factors), and differentiating factors (*for example,* colony stimulating factors (CSF)]. Numerous other proteins which have both pharmaceutical and industrial applications are produced by cell culture.

In general, cytokines and other proteins are produced by either purifying the natural protein from cell culture or recombinantly producing the protein in insect, microbial or human cells. Natural cytokines and other proteins are preferable in that they are known to contain the full repertoire of native forms of a given cytokine or protein and have the proper structure, but they are expensive and time-consuming to produce.

Recombinantly produced cytokines and other proteins are less expensive to make, but dependent upon the source may contain foreign antigens, resulting in an immune response by the subject to which they are administered, or may be less active due to structural variation from the native form, i.e., glycosylation pattern.

Thus, a method for enhancing the production of natural cytokines and other proteins to make them less expensive to produce would be advantageous.

Present methods utilize expression of these factors in microbial systems, which may not permit the proper glycosylation for native folding of the proteins, or in human cells with low production levels.

One exemplary group of cytokines, the interferons are produced in response to viral infections or growth of tumor cells. These glycoproteins possess antitumor and immunomodulatory activities in addition to their antiviral effects. Since 1994, IFNs have received FDA approval for specific clinical indications in the United States. Recently, two preparations of IFN-beta, one produced in *E. coli* and the other in Chinese hamster ovarian cells, have been approved for patients with multiple sclerosis. The former product has been known to induce of anti-IFN antibodies, and thus the formation of interferon immune complexes. It also causes undesirable effects including injection site tissue necrosis in most patients. Additional deficiencies have been attributed to bacterially-produced IFNs, including the induction of antibodies, probably due to lack of glycosylation; and limited efficacy of IFN-alpha in various diseases may be attributed, in part, to lack of other subtypes in the recombinant formulations. Previous studies have shown that the incidence of rejection as reflected by antibody formation can be as high as 20 to 38% for bacterially-produced IFN compared with only 1.2% for natural IFN-alpha (Antonelli, *et al.,* 1991; Antonelli, *et al.,* 1997).

dsRNA-activated protein kinase (PKR) referred to as P1/e1F2 kinase, DAI or dsI for dsRNA-activated inhibitor, and p68 (human) or p65 (murine) kinase, is a serine/threonine kinase whose enzymatic activation requires binding to dsRNA or to single-stranded RNA presenting internal dsRNA structures and consequent autophosphorylation (Galabru and Hovanessian, 1987; Meurs, *et al.,* 1990). PKR play a key role in the expression of a number of useful cytokines including interferons, as described in WO 97/08324, expressly incorporated by reference herein.

Activities attributed to PKR include a role in (1) mediating the antiviral and anti-proliferative activities of IFN-alpha and IFN-beta, (2) the response of uninfected cells to physiologic stress, and (3) cell growth regulation (Clemens and Elia, 1997; Zamanian-Daryoush, *et al.,* 1999).

The best characterized *in vivo* substrate for PKR is the alpha subunit of eukaryotic initiation factor-2 (eIF-2a) which, once phosphorylated, ultimately leads to inhibition of cellular and viral protein synthesis (Hershey, J.W.B., 1991). PKR has been demonstrated to phosphorylate initiation factor e1F-2 alpha *in vitro* when activated by double-stranded RNA (Chong, *et al.,* 1992).

It has also been suggested that PKR may function as a tumor suppressor and inducer of apoptosis. (See, *for example,* Clemens and Bommer, 1999; Koromilas, *et al.,* 1992), with recent results indicating that expression of an active form of PKR triggers apoptosis, possibly through upregulation of the Fas receptor (Donze, O., *et al.,* 1999). See, also Yeung, M.C., *et al.,* 1996; Yeung, M., and Lau, A.S., 1998).

It would be desirable to inhibit apoptotic cell death in cultured cell Lines, as a means to prolong and thereby enhance the production of cytokines and other proteins by the cells in such cultures.

The invention includes, in one aspect, a method for producing a selected cytokine or cytokines in a human cell culture. The method comprises culturing a human cell line capable of producing cytokines and transfected with a first vector containing DNA encoding a protein effective to inhibit cell apoptosis under the control of a first promoter and a second vector containing DNA encoding double-stranded-RNA-dependent-kinase (PKR) under the control of a second promoter. The cells are cultured under conditions in which PKR is overproduced in the transfected cells, as evidenced by levels of PKR in the transfected cell line which are higher than those obtained in the human cell line which is not transfected with the first and second vectors, when grown under the same culture conditions. The PKR-overproducing cells are treated to induce cytokine, e.g., by exposing the cells to double-stranded RNA (dsRNA), and the cytokine(s) produced by the cultured, treated cell line are collected.

The cultured cells are preferably prepared by transfecting a human cell capable of producing cytokines successivefully with the first vector and the second vector. The protein effective to inhibit apopotosis may be, for example, Bcl-2a, Bcl-X_{L}, a modified form of eukaryotic translation initiation factor 2 alpha (eIF-2 alpha) and eukaryotic translation initiation factor (eIF-3), a modified form of Fas-associated death domain (FADD), a modified form of Bcl-Xₛ, a modified form of Bcl-2-homologous antagonist/killer (BAK) and a modified from of BAX, preferably Bcl-2a or Bcl-X_{L}

The first and or second promoter may be inducible, e,g., a metallothionein promoter. The cytokine(s) produced may be one or more of the following: interferons, including IFN-gamma, IFN-alpha and IFN-beta; tumor necrosis factors (TNF), including TNF-alpha, TNF-beta and TNF soluble receptors (sTNF-R); interleukins (IL), including IL-2, IL-3, IL-4, IL-5, IL-6, IL-7, IL-8, IL-11 and IL-12; colony stimulating factors, including granulocyte colony stimulating factors (G-CSF) and granulocyte-macrophage colony stimulating factor (GM-CSF); angiogenic factors, including fibroblast growth factor (FGF), vascular endothelial growth factor (VEGF); platelet-derived growth factors 1 and 2 (PDGF 1 and 2); chemokines, including Regulated Upon Activation Normally T-Expressed Secreted (RANTES); macrophage inflammatory proteins (MIP), such as MIP-1alpha and MIP-2alpha, monocyte chemotactic protein-1(MCP); anti-angiogenic factors, including angiostatin and endostatin; leukemia inhibitory factor (LIF); ciliary neurotrophic factor, cardiotrophin and oncostatins, including oncostatin M.

The human cell is derived, for example, from human fibroblasts or immune cells, B cells, T cells, monocytes, neutrophils, natural killer cells, pro-monocytic U937 cells, Namalwa cells, MRC-5 cells, WI-38 cells, Flow 1000 cells, Flow 4000 cells, FS-4, FS-7 cells, MG-63 cells, CCRF-SB cells, CCRF-CEM, Jurkat cells, WIL2 cells and THP-1 cells.

In another aspect, the invention includes a method for producing cytokines in a human cell culture by culturing a human cytokine-producing cell under conditions of PKR overproduction and cytokine induction. The method includes, for increasing the viability of the cells, the use as the cell line of cells which have been transfected with a vector containing DNA encoding a protein effective to inhibit apoptosis in the cells.

A preferred cell line is one that has been transfected with a vector containing DNA expressing PKR. The DNA encoding the protein effective to inhibit apoptosis in the cells encodes, for example, Bcl-2, Bcl-X_{L}, a modified form of eukaryotic translation initiation factor 2 alpha (eIF-2 alpha) or eukaryotic translation initiation factor (eIF-3), a modified form of Fas-associated death domain (FADD), a modified form of Bcl-X_{S}, a modified form of BAK and a modified form of BAX, preferably Bcl-2 or BcL-X.

These and other objects and features of the invention will become more fully apparent when the following detailed description of the invention is read in conjunction with the accompanying drawings.
Figures 1A and 1B show the vectors, pEF-FLAG-Bcl-X_{L} and pcDNA-Flag-PKR, respectively, useful in practicing the invention;
Figures 2A and 2B show the percentage of viable 6A, A9 and WT cell lines following cytokine induction by Sendai virus and poly IC, respectively; and
Figures 3A and 3B show the IFN-alpha levels produced in 6A, A9 and WT cell lines following treatment with Sendai virus and poly IC, respectively.

### I. Definitions

The term "vector" refers to a nucleotide sequence that can assimilate new nucleic acids, and propagate those new sequences in an appropriate host. Vectors include, but are not limited to recombinant plasmids and viruses. The vector (*for example,* plasmid or recombinant virus) comprising the nucleic acid of the invention can be in a carrier, for example, a plasmid complexed to a protein, a plasmid complexed with lipid-based nucleic acid transduction systems, or other non-viral carrier systems.

A cloning or expression vector may comprise additional elements, for example, the expression vector may have two replication systems, thus allowing it to be maintained in two organisms, for example in human or insect cells for expression and in a prokaryotic host for cloning and amplification.

Both cloning and expression vectors contain a nucleic acid sequence that enables the vector to replicate in one or more selected host cells. Such sequences are well known for a variety of bacteria, yeast, and viruses. Further, for integrating expression vectors, the expression vector contains at least one sequence homologous to the host cell genome, and preferably two homologous sequences that flank the expression construct. The integrating vector may be directed to a specific locus in the host cell by selecting the appropriate homologous sequence for inclusion in the vector. Constructs for integrating vectors are well known in the art.

Cloning and expression vectors will typically contain a selectable marker. Typical selectable marker genes encode proteins that (a) confer resistance to antibiotics or other toxins, *for example,* ampicillin, neomycin, methotrexate, or tetracycline, (b) complement auxotrophic deficiencies, or (c) supply critical nutrients not available from complex media, *for example,* the gene encoding D-alanine racemase for *Bacilli.*

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

The nucleic acid coding sequence must be "operably linked" by placing it in a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" DNA sequences are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

Promoter sequences encode either constitutive or inducible promoters. The promoters may be either naturally occurring, engineered or hybrid promoters.

As used herein, the term "PKR expression" refers to transcription and translation of PKR gene, the products of which include precursor RNA, mRNA, polypeptide, post-translation processed polypeptide, and derivatives thereof, and including PKRs from other species such as murine or simian enzymes. By way of example, assays for PKR expression include autophosphorylation assays, assay for eIF2α phosphorylation, Western and Northern blot analysis and reverse transcriptase polymerase chain reaction (RT-PCR) for PKR mRNA.

As used herein, the terms "biological activity of PKR" and "biologically active PKR" refer to any biological activity associated with PKR, or any fragment, derivative, or analog of PKR, such as enzymatic activity, specifically including autophosphorylation activity and eukaryotic translation initiation factor 2 (eIF-2) phosphorylation activity.

As used herein, the terms "normal level of PKR activity" and "normal level of PKR expression" refer to the level of PKR activity or expression, determined to be present in unstimulated or uninfected cells of a particular type, *for example,* a particular cell line. It will be appreciated that such "normal" PKR activity or expression, is reported as a range of PKR activity or expression which is generally observed for a given type of cells that have not been transfected with a vector encoding PKR, are unstimulated (not induced or primed) and uninfected.

The range of "normal" PKR activity or expression may vary somewhat dependent upon culture conditions. For example, the U937 cell line may have a normal range of PKR activity which differs from the normal range of PKR activity for the Vero or Namalwa cell lines. It follows that over-expression of PKR means an expression level which is above the normal range of PKR expression generally observed for a given type of cells which are not transfected with a vector encoding PKR, unstimulated (not induced or primed) and uninfected. Accordingly, "overexpression" of PKR means a range of PKR activity or expression which is greater than that generally observed for a given type of cells which are not transfected with a vector encoding PKR, unstimulated (not induced or primed) and are uninfected.

Similar definitions apply to Bcl-2, Bcl-X_{L} and related homologues, wherein "overexpression" of Bcl-2 or Bcl-X_{L}, respectively means a range of Bcl-2 or Bcl-X_{L} activity or expression which is greater than that generally observed for a given type of cells which are not transfected with a vector encoding Bcl-2 or Bel-X_{L}, and have not been stimulated to undergo apoptosis.

As used herein, the term "modified form of", relative to proteins associated with apoptosis, exemplified by, eIF-2a or eIF-2alpha, eIF-3, FADD, Bcl-X_{S}, BAK, BAX, etc., means a derivative or variant form of the native protein. That is, a "modified form of" a protein has a derivative polypeptide sequence containing at least one amino acid substitution, deletion or insertion, with amino acid substitutions being particularly preferred. The amino acid substitution, insertion or deletion may occur at any residue within the polypeptide sequence, which interferes with the biological activity of the protein. The corresponding nucleic acid sequence which encodes the variant or derivative protein is considered to be a "mutated" or "modified form of" the gene or coding sequence therefor, and is included within the scope of the invention.

As used herein, the terms "biological activity" or "biologically active", refer to the activity attributed to a particular apoptosis-associated protein in a cell line in culture, in its native form. It will be appreciated that the "biological activity" of such a protein may vary somewhat dependent upon culture conditions and is generally reported as a range of activity. Accordingly, a "biologically inactive" form of a protein refers to a form of the protein which has been modified in a manner which interferes with the activity of the protein as it is found in nature. For example, a "biologically inactive" form of eIF-2a may be a form of the protein which has a modified phosphorylation site, which does not act as a protein synthesis inhibitor and does not exhibit contribute to apoptosis as does the native "biologically active" form of eIF-2a.

As used herein, the terms "normal level of cytokine" and "normal level of protein", relative to activity, expression, and production, refer to the level of cytokine or other protein activity, expression or production, determined to be present in cells of a particular type which have not been treated in a manner effective to inhibit apoptosis and have not been transformed in a manner effective to result in PKR overexpression. Examples include, a cell line which has not been transfected with a transgene which encodes PKR or a protein associated with apoptosis and which either normally produces or is capable of producing a given cytokine or other protein. It will be appreciated that such "normal" cytokine or other protein activity, expression, or production, is reported as a range of activity, expression, or production, which is generally observed for a given type of cells and may vary somewhat dependent upon culture conditions.

Similarly, the definitions that apply to cytokines, also apply to "other proteins", produced by the methods of the invention.

For example, a given cell line which does not overexpress PKR, and has not been treated in a manner effective to inhibit apoptosis, has a normal range of cytokine activity which differs from the range of cytokine activity for that same cell line following modification which results in (1) over-expression of PKR, and (2) inhibition of apoptotic cell death.

The terms "apoptotic cell death", "programmed cell death" and "apoptosis", as used herein refer to any cell death that results from, or is related to, the complex cascade of cellular events that occur at specific stages of cellular differentiation and in response to specific stimuli. Apoptotic cell death is characterized by condensation of the cytoplasm and nucleus of dying cells.

As used herein, the term "inhibit apoptotic cell death", means to partially or completely inhibit the cell death process over the time period a cell line is cultured for the purpose of cytokine or other protein expression. Such inhibition generally means the amount of apoptotic cell death is decreased by at least 20% and preferably 80% or more relative to the amount of apoptotic cell death observed in a cell line which has not been modified in a manner effective to inhibit apoptosis.

In the case of cytokine production, such inhibition generally means the amount of apoptotic cell death is decreased by at least 50% and preferably 80% or more relative to the amount of apoptotic cell death observed in a PKR-overexpressing cell line which has not been modified in a manner effective to inhibit apoptosis.

### II. PKR

IFNs elicit their biological activities by binding to their cognate receptors followed by signal transduction leading to induction of IFN-stimulated genes, ISG. These ISG mediate the biological activities of IFNs by at least two pathways intracellularly: degradation of RNA via the activation of a specific ribonuclease, and induction of an IFN-regulated and double stranded RNA-activated kinase (PKR).

Examples of ISGs include PKR (formerly known as p68 kinase), 2'-5'-linked oligoadenylate (2-5A) synthetase, and Mx proteins (Taylor and Grossberg, 1990; Williams, 1991, 1997). The 2-5A synthetase, using ATP as substrate, synthesizes short oligomers of up to 12 adenylate residues linked by 2'-5'-phosphodiester bonds. The resulting oligoadenylate molecules activate a latent ribonuclease, RNase L, that degrades viral and cellular RNAs. The 2-5A synthetase pathway appears to be important for (1) a reduction in the synthesis of viral proteins in cell-free protein-synthesizing systems isolated from IFN-treated cells and (2) inhibition of tumor cell growth.

PKR is the only identified dsRNA-binding protein known to possess a kinase activity. PKR is a serine/threonine kinase whose enzymatic activation requires dsRNA binding and consequent autophosphorylation (Meurs, *et al.,* 1990; Feng GS *et al,* 1992).

Various functions have been attributed to PKR, including, phosphorylation of eukaryotic initiation factor-2 (eIF-2alpha), which, once phosphorylated, leads to inhibition of protein synthesis (Hershey, *et al.,* 1991). This particular function of PKR has been suggested as one of the mechanisms responsible for mediating the antiviral and anti-proliferative activities of IFN-alpha and IFN-beta. An additional biological function for PKR is its putative role as a signal transducer, *for example,* by can phosphorylation of IkB, resulting in the release and activation of nuclear factor kB (NF-kB) (Kumar A *et al.,* 1994).

It has previously been demonstrated that PKR mediates the transcriptional activation of IFN expression (Der D and Lau AS, 199). Consistent with this observation, suppression of endogenous PKR activity by transfecting U937 cells with antisense to PKR or expression of a PKR-deficient mutant resulted in diminished induction of IFN in response to viral infection (Der D and Lau AS, 1995).

In summary, PKR has been associated with (1) signal transduction for complex receptor systems (including IFN, TNF and Fas), (2) transcriptional activation of cytokine genes, (3) initiation of apoptosis, and (4) inhibition of protein synthesis by phosphorylating eIF-2α.

In accordance with the present invention, it has been discovered that cell viability is increased in cells producing cytokines under conditions of PKR overexpression by employing, as the cytokine-producing cell line, cells which have been transfected with a gene encoding a protein that is capable of inhibiting apoptosis in the cells, under the control of a suitable promoter. The promoter may be a constitutive promoter or one which is inducible by addition to the culture medium of a suitable inducer, such as a metallotheinein promoter that can upregulated by addition of certain metal salts. PKR overproduction is achieved, in a preferred embodiment of the invention, using cells that have also been transfected with a gene encoding PKR, also under the control of a suitable promoter, either constitutive or inducible, for PKR overproduction in culture.

Examples 1 and 2 herein describe exemplary vectors and transfection methods for obtaining cells suitable for use in the invention. Typically, the cells are first transfected with the vector containing the anti-apoptotic gene, then successful transformants are further transfected with the vector containing the PKR gene. This allows for the second transfection and selection to be carried out with cells that have already been "stabilized" with an anti-apoptopic function. The vector construction and tranfection conditions are conventional, and known to those skilled in the art. In particular, it is well known, in such vector constructions, to obtain suitable plasmids or other vectors, e.g., from commercial sources, capable of being introduced into and replicating within selected human cells, where the plasmids may also be equipped with selectable markers, insertion sites, and suitable control elements, such as termination sequences. The plasmid may or may not have its own promoter. If not, the vector construction will require insertion of a suitable promoter, sequences of which are widely available, and can be obtained for example, from the GenBank database of coding sequences. Typical coding sequences for a PKR gene, and for a Bcl-X_{L} gene are referenced in Example 1, and can be obtained from the GenBank as cited. A variety of genes whose expression products are known to inhibit apoptosis may be employed, and are given below. The promoter and coding sequences are inserted into a suitable vector according to well-known recombinant techniques.

### III. APOPTOSIS

Apoptosis or programmed cell death is a cell-intrinsic suicide process (reviewed in Orrenius 1995; Stellar 1995; Vaux 1993). Apoptosis provides many advantages for organisms, both during fetal development (Cohen 1992), in controlling the formation of organs (Nagata & Suda 1995; Vaux 1993), and for purposes of homeostasis in adult life. Once committed to apoptosis, the cells undergo new rounds of protein synthesis and various morphological/physiological changes including cytoplasmic condensation, nuclear chromatin condensation, membrane blebbing, and eventual DNA degradation, detected as a characteristic oligonucleosomal ladder (Levine AJ, 1993). The dying cell eventually fragments into membrane-bound apoptotic bodies that are rapidly phagocytosed and digested by macrophages or by neighboring cells.

Apoptosis serves as a defense mechanism to remove unwanted and potentially dangerous cells including virus-infected cells, self-reactive lymphocytes in autoimmune diseases, or malignant cells (Oehm, *et al.,* 1992; Yonehara, *et al,* 1989; Vaux, 1993). Apoptosis has been implicated as a means to minimize the risk of cancer cell development in tissues frequently exposed to mutagenic chemicals, carcinogens, or UV radiation.

Unlike the morphological transformation events associated with apoptosis, the genetics and mechanisms involved in programmed cell death are not as well understood.

A further protection against malignancy is afforded by TNF-α, a proinflammatory cytokine, produced in response to activation of the immune system, and which can trigger the apoptotic death of transformed host cells (Heller, 1992, Yeung, 1996).

Deregulation of the apoptosis process may contribute to the pathogenesis of disease processes (Thompson, 1995). It is believed to play a critical role for disease development including cancer, AIDS, ischemic stroke and neurodegenerative disorders, and evidence suggests that both inhibition of cell death and inappropriate cell death may be deleterious to the host. For example, neurodegenerative diseases including Alzheimer and Parkinson diseases are associated with the premature death of particular subtypes of neurons (Kosik KS, 1992), while inappropriate suppression or inherent deficiency of cellular apoptosis may result in the malignant transformation of cells (Korsmeyer, 1992).

Individual proto-oncogenes have also been associated with apoptosis in the expression in cells undergoing apoptosis and in the affect of the modulation of a individual proto-oncogenes on the process. The list of proto-oncogenes implicated include c-myc, Fas (APO-1), p53, and Bcl-2 in addition to other genes such as ced-3, ced-4, ced-9 and Ice, initially identified in early studies on *C. elegans* (Stellar, 1995; Cohen, 1993). Coding sequences of the proteins can be found, for example, in the GenBank database.

### THE ROLE OF PKR AND TNF-α IN APOPTOSIS

TNFs, as prototypes proinflammatory cytokines, are cytotoxic proteins produced by activated immune cells during the processes of pathogen elimination, antiviral activities, and tumor destruction. However, high levels of TNF*-*alpha *in vivo* can be detrimental since TNF-alpha induces metabolic disturbances, wasting, and suppression of hematopoiesis. At the cellular level, TNF-alpha induces production of superoxide radicals, activation of lysosomal enzymes (Larrick, *et al.,* 1990; Liddil, *et al.,* 1989), and fragmentation of DNA by the activation of endonuclease activity (Rubin, *et al.,* 1988), leading to apoptosis.

The exact mechanism of TNF-α-associated apoptosis is unclear, and various mechanism have been proposed. (See, *for example,* Dressler, *et al.,* 1992; Obeid, *et al.,* 1993). It has been shown that: (i) TNF-α treatment results in the activation of several serine/threonine protein kinases including PKR; (ii) TNF-α and PKR mobilize NF-kB; (iii) PKR is a serine/threonine protein kinase and is growth-inhibiting; iv) PKR plays a pivotal role in the TNF-α signaling pathway, and v) tumor suppressor gene p53 plays a role in the TNF-α-induced apoptosis process. (See, Guy, *et al.,* 1992; Van Lint, *et al.,* 1992, Yeung and Lau, *et al.,* 1996).

### IV. MODULATED EXPRESSION OF CELLULAR FACTORS

The invention provides methods for enhanced production of cytokines in human cell culture by suppressing the apoptotic cell death process. By inhibiting apoptosis, the cell lines described herein have a longer lifespan in culture; as a consequence, biosynthesis of cytokines is increased and/or the time over which the cells function to produce cytokines is increased.

Suppression of apoptotic cell death process in human cell culture may be achieved by any of a number of strategies directed to inhibition of apoptosis, including: (1) overexpression of an anti-oncogene, such as Bcl-2 (GenBank Accession No. M 14745), Bcl-X_{L} (GenBank Accession No. L20121) or its homologue (2) suppression of endogenous FADD (GenBank Accession No. NM 00384) activity, *for example,* overexpression of a mutant form of FADD, mutation of the endogenous FADD gene by homologous recombination or site directed mutagenesis; (3) suppression of eIF2-alpha (GenBank Accession No. A 457497) phosphorylation, for *example,* by overexpression of a mutant form of eIF2-alpha, by mutation of the endogenous eIF2-alpha gene by homologous recombination or site directed mutagenesis, thereby inhibiting the downstream substrates of PKR; or (4) use of a transdominant mutant, by mutation of an endogenous gene for one or more pro-apoptotic counterparts of Bcl-2, *for example* BAX (GenBank Accession No. L22473), BAK (GenBank Accession No. BE221666), and Bcl-X_{S} (GenBank Accession No. L20122) by homologous recombination or site-directed mutagenesis, or by gene ablation or gene deletion of one or more of BAX, BAK, and Bcl-X_{S}.

Cell death may be detected by staining of cells with propidium iodide (PI), or by use of assays specific to apoptotic cell death, for *example* staining with annexin V (Vermes, *et al.,* 1995). Necrotic cell death may be distinguished from apoptotic cell death by evaluating the results of a combination of the assays for cell viability, together with microscopic observation of the morphology of the relevant cells.

### INHIBITION OF APOPTOSIS

In one aspect, the invention provides a method for modulating cytokine or other protein production by modifying the cells within the cell culture in a manner effective to result in partial suppression of, or delay in, cell death process, by culturing a particular cell line under conditions resulting in such that above-normal levels of cytokine or other protein production are achieved relative to a culture of that same cell line which does not have the suppression of the apoptotic cell death process under the same conditions.

The invention further provides a method of producing a cytokine or other protein, comprising culturing a host cell transfected with an expression vector having a promoter which functions in the host cell, operably linked to a DNA sequence encoding a desirable gene which is effective to inhibit apoptosis. These proteins maybe, for example, Bcl-2a, Bel-X_{L}, a modified form of eukaryotic translation initiation factor 2 alpha (eIF-2 alpha) or eukaryotic translation initiation factor (eIF-3) (GenBank Accession No. BE221666), a modified form of Fas-associated death domain (FADD), a modified form of Bcl-X_{S} (GenBank Accession No.L20121), a modified form of BAK and a modified from of BAX, preferably Bcl-2a or Bcl-X_{L}. The desirable gene is overexpressed in the host cell resulting in a suppression or delay in apoptotic cell death. Additional means to effect suppression of endogenous gene expression may be employed, including, but not limited to, mutation of the endogenous gene, *for example,* by homologous recombination or site directed mutagenesis, gene deletion or gene ablation.

As noted above, cells containing these genes are typically co-transformants also containing vectors with exogenous PKR gene, for achieving PKR-overexpression in the cells. Human cell lines suitable for use in the invention include fibroblasts or immune cells, B cells, T cells, monocytes, neutrophils, natural killer cells, pro-monocytic U937 cells, Namalwa cells, MRC-5 cells, WI-38 cells, Flow 1000 cells, Flow 4000 cells, FS-4, FS-7 cells, MG-63 cells, CCRF-SB cells, CCRF-CEM, Jurkat cells, WIL2 cells and THP-1 cells.

In a further embodiment of the invention, cells treated to inhibit apoptosis include cell lines generasslly used to express a given cytokine or protein of interest, wherein the expression of the protein is not associated with PKR, for example, CHO (Chinese hamster ovary) cells.

### MODIFIED FORMS OF APOPTOSIS-ASSOCIATED PROTEINS

As set forth above, apoptosis may be inhibited by decreasing the expression of proteins associated with facilitating the apoptotic process in nature, by modifying cells in a manner effective to express modified or variant forms of such proteins. Alternatively, apoptosis may be inhibited by increasing the expression of proteins associated with blocking the apoptotic process in nature.

In a preferred embodiment, the modified eIF-2a, modified FADD, modified Bcl-X_{S} modified BAK and modified BAX proteins are derivative or variant eIF-2a, FADD, Bcl-X_{S}, BAK and BAX forms of the respective proteins as they are found in nature. That is, the derivative polypeptide or protein contains at least one amino acid substitution, deletion or insertion, with amino acid substitutions being preferred. The amino acid substitution, insertion or deletion may occur at any residue within the amino acid sequence of the polypeptide or protein, as long as it interferes with the biological activity of the protein.

These modified or variant forms of such native proteins ordinarily are prepared by site specific mutagenesis of nucleotides in the DNA encoding the eIF-2a, FADD, Bcl-X_{S}, BAK or BAK protein, using cassette or PCR mutagenesis or other techniques well known in the art, to produce DNA encoding the variant, and thereafter expressing the DNA in recombinant form in cell culture.

Site-specific mutagenesis provides a means for introducing one or more nucleotide sequence changes into the DNA encoding a given protein, and in general, the technique of site-specific mutagenesis is well known in the art, and typically employs a phage vector which exists in both a single stranded and double stranded form.

It will be understood that all mutant, modified or variant forms of native proteins described herein can be created by point or site directed mutagenesis of the appropriate nucleic acid sequence, or by homologous recombination (knock-in or knock-out) to accomplish inhibition of function or activity of the target gene or its protein.

In general, cDNA sequences for both yeast and human genes encoding modified forms of the eIF-2a, FADD, Bcl-X_{S}, BAK or BAK protein are inserted into an expression vector under the control of a strong constitutive viral promoter (the CMV promoter or the SV40 promoter). In other cases, the cDNA sequence is inserted into an expression vector under the control of an inducible promoter, for example, a metallothionein promoter. Selectable markers for use in such expression vectors are generally known in the art, for example, neo (G418, geneticin) and EcoGPT (mycophenolic acid). It will be understood that the expression vectors further contain components necessary to facilitate expression in a given cell type, and which are generally known in the art.

Cells are transfected using standard procedures including electroporation, calcium phosphate, DEAE dextran, lipofection, or Lipofectamine treatment, and selected in the appropriate antibiotic. Procedures for the cloning and expression of modified forms of native protein using recombinant DNA technology are generally known in the art, as described in Ausubel, *et al.,* 1992 and Sambrook, *et al.,* 1989, expressly incorporated by reference, herein.

In one approach cells for cytokine production may be co-transfected with a nucleic acid construct or expression vector effective to express a modified form of eIF-2a, FADD, Bcl-X_{S}, BAK or BAK and an expression vector effective to overexpress PKR. In a related approach, a PKR-overexpressing cell line may be transfected with a nucleic acid construct or expression vector effective to express a modified form of eIF-2a, FADD, Bcl-X_{S}, BAK or BAK.

In another aspect of the invention, cells for the production of proteins whose expression is not regulated by PKR are transfected with a nucleic acid construct or expression vector effective to express a modified form of eIF-2a, FADD, Bcl-X_{S}, BAK or BAK.

Following transfection and selection of transformed cells, the cells are further cultured in manner effective to result in production of the cytokine or other protein of interest, as further described below.

### SUPPRESSING eIF2alpha PHOSPHORYLATION

It has been demonstrated that PKR plays a critical role in the TNF-induced and p53-mediated apoptosis in cells including promonocytic U937 cells (Yeung, M.C., *et al.,* 1996: Yeung, M., and Lau, A.S., 1998). Suppression of PKR activity, by transfecting U937 cells with PKR-antisense or PKR-mutant expression plasmids, renders the cells more resistant to TNF or endotoxin induced cytotoxicity. Since eIF-2alpha is a physiological substrate of PKR, its phosphorylation by PKR has been shown to be sufficient to induce apoptosis.

Consistently, TNF-induced apoptosis has been correlated with increased phosphorylation of the alpha subunit of the eIF-2 (Srivastave, *et al.,* 1998).

As set forth above, eIF-2alpha contributes to the inhibition of cellular and viral protein synthesis following phosphorylation. It follows that suppression of PKR-mediated phosphorylation of eIF-2alpha, by mutating the phosphorylation site of the factor, provides a means to inhibit the apoptotic affect of PKR overexpression on cultured cell lines.

A variant eIF-2aplha protein has been expressed in lymphoid cells, using a vector containing the coding sequence for a modified form of eIF-2aplha.

The yeast and human eIF-2alpha genes were mutated using a modified DNA sequence encoding a polypeptide having a single amino acid change at position 59, resulting in a serine to alanine variant. Position 59 has previously been shown to be the phosphoresce residue phosphorylated by PKR. The alanine variant is not inactive, but is insensitive to the effects of PKR.

Amplification of the eIF-2a and PKR expressing plasmids was accomplished using MTX selection in the presence of a DHFR expression cassette contained on the same plasmid as the PKR cassette. This results in the expression of a DHFR mutant capable of greater amplification than endogenous DHFR, enabling selective increases of the co-expressed product.

A variant (mutated) eIF-2alpha cDNA sequence was inserted into a vector effective to express the inserted fragment under the control of a strong viral promoter, as described above.

Cells expressing a modified form of eIF-2a were generated, selected, further cultured in manner effective to result in production of the cytokine or other protein of interest, and analyzed for the biosynthesis of the cytokine or other protein of interest, as described below.

### SUPPRESSING ENDOGENOUS FADD ACTIVITY

The Fas receptor is a member of the TNF and the nerve growth factor receptor superfamily (Stellar, 1995). Following binding of Fas ligand to the Fas receptor, apoptosis is initiated via immediate downstream effectors, including FADD, FLICE, and TRADD. FADD is a cytoplasmic protein with a death domain which is crucial for CD 95 ligand and TNF induced apoptosis.

The binding of these proteins to their respective receptors results in activation of the caspase protease cascade and facilitates apoptosis. It has been previously demonstrated that Fas expression and consequent apoptosis are regulated by PKR activity in NIH-3T3 cells (Donze, *et al.,* 1999). In cells transfected with a transdominant negative mutant deficient in PKR kinase activity, the expression of Fas, TNFR-1, FADD (Fas-associated death domain), FLICE, Bad and BAX are suppressed, and the cells were resistant to apoptosis-inducing agents. Additionally, murine fibroblasts lacking FADD were almost resistant to dsRNA-mediated cell death (Balachandran, *et al.,* 1998).

Variant, non-functional human and murine FADD genes were generated from the wild type FADD gene (Chinnaiyen, *et al.,* 1995; Yeh, *et al.,* 1998). Mutant genes have been used to generate murine FADD-/- cells that were deficient in FADD activity with consequent resistance to PKR-mediated cytotoxicity (Balachandran, *et al.,* 1998). The Fas-mediated cell death process is inhibited or eliminated in cells expressing a modified FADD gene, allowing for inhibition of apoptosis. The inhibitory effect of such a biologically inactive form of FADD is not circumvented by PKR activation.

A mutated FADD cDNA sequence was inserted into a vector effective to express the inserted fragment under the control of a strong viral promoter, as described above.

Cells expressing a modified form of FADD were generated, selected, further cultured in manner effective to result in production of the cytokine or other protein of interest, and analyzed for the biosynthesis of the cytokine or other protein of interest, as described below.

### OVEREXPRESSION OF Bcl-2, Bcl-X_{L}, OR ITS HOMOLOGUE

The Bcl-2 family of gene products is commonly involved in apoptotic processes that are previously studied in diverse biological systems. Bcl-2a and Bel-X_{L} are considered to be anti-apoptotic proteins (Boise and Thompson, 1995; Schendel, 1998) and previous studies on lymphocytic and myeloid cells have indicated a role for Bcl-2a in the maintenance of cell growth and the prevention of cell death (Cohen, 1993). Additionally, Bcl-2a plays a significant role in prevention of neuronal cell apoptosis (Garcia, *et al.,* 1992), probably by decreasing the generation of reactive oxygen species (Kane, *et al.,* 1993).

The viability of many cells is dependent on a constant or intermittent supply of cytokines or growth factors. In the absence of such cytokines or growth factors, the cells under go apoptosis. The Bcl-2 family of proteins are integral to the apoptotic process mediated by cytokines. Over-expression of Bcl-2 and Bel-X_{L} has been shown to suppresses apoptosis when cytokines are withdrawn. Over-expression of BAX, and BAK has been shown to override the incoming signals from the cytokine receptors and induce apoptosis.

In one exemplary application of the invention, Bcl-2 overexpressing cells were produced by transfecting a target cell line with the pSV-2-Bcl2 expression plasmids (Reed, *et al.,* 1988; Reed, *et al.,* 1981).

Bcl-2 and Bel-X_{L} overexpressing cells are generated, selected, further cultured in manner effective to result in production of the cytokine or other protein of interest, and analyzed for the biosynthesis of the cytokine or other protein of interest, as described below.

### INHIBITING PRO-APOPTOTIC COUNTERPARTS OF Bcl-2

In general, BAX, BAK, Bcl-X_{S} and others are pro-apoptotic proteins (Boise and Thompson, 1998). As set forth above, overexpression of BAX, BAK, Bcl-X_{S} has been shown to override the incoming signals from cytokine-mediated signaling associated with cell viability and to induce apoptosis.

Accordingly, variant, non-functional human BAX, BAK, Bcl-X_{S} genes may generated from the wild type BAX, BAK, Bcl-X_{S} genes. Such mutant genes may be used to generate transformed cells deficient in BAX, BAK, or Bcl-X_{S} activity, respectively, allowing for inhibition of apoptosis. The inhibitory effect of such the biologically inactive form of BAX, BAK, or Bcl-X_{S} on apoptosis provides a means to circumvent the stimulatory effect of PKR overexpression on apoptotic cell death in cultured cell lines.

A mutated or variant human BAX, BAK, or Bcl-X_{S} cDNA sequence may be inserted into a vector effective to express the inserted fragment under the control of a strong viral promoter, as described above.

Cells expressing a modified form of human BAX, BAK, or Bcl-X_{S} are thereby generated, selected, further cultured in manner effective to result in production of a cytokine or other protein of interest, and then analyzed for the biosynthesis of the cytokine or other protein of interest, as described below.

### V. CYTOKINES

Cytokines elicit their biological activities by binding to their cognate receptors followed by signal transduction leading to stimulation of various biochemical processes. In some cases, the expression of such receptors is regulated by specific signals, for example a cytokine may be involved in positive or negative feedback loops and thereby regulate the expression of the receptor for the same or a different cytokine. Such receptors may be the same type of cell that produces the cytokine or a different type of cell.

Cytokines serve to mediate and regulate immune and inflammatory responses. In general, cytokine production is transient and production takes place during a short period of transcription resulting in production of mRNA transcripts which are also short-lived and subject to post-transcriptional control mechanisms. Recent studies have indicated that a common signal transduction pathway, the "Jak/STAT" pathway, is used by a variety of cytokines (Abbas, *et al.,* 1997).

It will be appreciated that the cellular source of cytokines is a distinguishing characteristic of each individual cytokine that may be produced by multiple diverse types of cells. In addition, a given cytokine (1) may act on more than one type of cells, (2) may have more than one effect on the same cell, (3) may have an activity shared with another cytokine, and (4) may influence the synthesis or effect of other cytokines, *for example,* by antagonizing, or synergizing the effects thereof.

The cytokine(s) produced may be one or more of the following: interferons, including IFN-gamma, IFN-alpha and IFN-beta; tumor necrosis factors (TNF), including TNF-alpha, TNF-beta and TNF soluble receptors (sTNF-R); interleukins (IL), including IL-2, IL-3, IL-4, IL-5, Il-6, Il-7, IL-8, IL-11 and IL-12; colony stimulating factors, including granulocyte colony stimulating factors (G-CSF) and granulocyte-macrophage colony stimulating factor (GM-CSF); angiogenic factors, including fibroblast growth factor (FGF), vascular endothelial growth factor (VEGF); platelet-derived growth factors 1 and 2 (PDGF 1 and 2); chemokines, including Regulated Upon Activation Normally T-Expressed Secreted (RANTES); macrophage inflammatory proteins (MIP), such as MIP-1alpha and MIP-2alpha; monocyte chemotactic protein-1 (MCP); anti-angiogenic factors, including angiostatin; endostatin leukemia inhibitory factor (LIF); ciliary neurotrophic factor; cardiotrophin and oncostatins, including oncostatin M.

The methods of the invention may also be used to increase the expression of any of a number of proteins which are capable of production in cell culture. Exemplary proteins include, but are not limited to, insulin, erythropoietin (EPO), tissue plasminogen activator (TPA), growth hormone and Factor VIII.

Once increased expression of a given cytokine or other protein is achieved, the cytokine or other protein thereby produced is purified from the cell culture. Exemplary procedures suitable for such purification include the following: antibody-affinity column chromatography, ion exchange chromatography; ethanol precipitation; reverse phase HPLC; chromatography on silica or on a cation-exchange resin such as DEAE; chromatofocusing; SDS-PAGE; ammonium sulfate precipitation; and gel filtration using, for example, Sephadex G-75. Various methods of protein purification may be employed and such methods are known in the art and described for example in Deutscher, 1990; Scopes, 1982. The purification step(s) selected will depend, for example, on the nature of the production process used and the particular cytokine or protein produced.

A "higher than normal level" of cytokine or other protein production means at least 200 or 300%, preferably 500% or more, of the cytokine or other protein production level for a given cell line in the absence of either transforming the cell line in a manner effective to result in over-expression of PKR or modifying the cell line in a manner effective to inhibit apoptotic cell death.

In the methods of the invention; it is preferred that a human cell line is modified by the combination of PKR overexpression and inhibition of apoptosis or inhibition of apoptosis alone, and cultured in a manner effective to enhance cytokine or other protein production respectively, by 10-1000 fold.

### VI. PKR OVEREXPRESSION, INHIBITION OF APOPTOSIS AND CYTOKINE PRODUCTION

A number of factors are known to be involved in the induction and/or enhanced expression of cytokines in cells, e.g., human cells. These factors include cytokine- and other protein-specific transcriptional regulators, for example interferon regulatory factors (IRF-1, LRF-3 and IRF-7), cytokine receptors, nuclear factor κB (NF-κB), activator protein-1 (AP-1), nuclear factor IL-6 (NF-IL6), and in particular PKR.

Enhancing the expression or activity of any of these factors will result in a higher than normal level of expression of the genes which encode one or more cytokines. Such enhanced expression of cytokine genes will result in more efficient and lower cost production of cytokines.

PKR, is used as herein as an example of a protein capable of regulating cytokine and other protein expression; however, it will be understood that other cytokine and other protein enhancing factors may be used in place of PKR, *for example,* 1) protein kinase C (PKC) inducers, TNF-α, GM-CSF, EGF and PDGF, G-CSF, TGF, TNF-alpha or TNF-beta, IL-1, IFNs (IFN-alpha, IFN-beta, IFN-gamma) or chemokines (IL-8, Macrophage inflammatory proteins [MIP-1a & -1b] and monocyte chemotactic proteins [MCPs]); 2) other cellular signaling factors such as PMA, calcium ionophores, sodium butyrate or endotoxin ; 3) polyI: C, double-stranded RNA or viral analog; 4) cellular stress signals that can activate PKR including heat shock or pathogen infections including virus), overproduces activated PKR and various cytokines.

By increasing the expression of PKR in a human cell, cytokine production can be increased. Animal cell cultures which express a higher than normal constitutive level of PKR or in which PKR expression can be induced to higher than normal levels are therefore useful for the production of cytokines.

The cells used to produce a given cytokine can overexpress PKR from any mammalian source, such as the PKR normally found in rabbit reticulocytes, various mouse tissues, or human peripheral blood mononuclear cells. Preferably murine p65 kinase and most preferably human p68 kinase is overexpressed, in a corresponding murine or human cell culture, respectively.

In some cases, the PKR which is overexpressed is an analog of PKR, *for example,* a non-natural protein kinase that can mediate dsRNA activation of cytokine and other protein transcription (usually obtained by modification of the gene encoding a native PKR protein).
Human cells capable of overexpressing PKR may be obtained by any number of methods, that are well known in the art or may be obtained from commercial sources.

Exemplary methods for obtaining PKR-overexpressing cells include selection for cells expressing higher than normal PKR levels, transfection with an expression vector encoding PKR under control of a promoter, or other methods which result in an increase in PKR expression over normal levels.

Appropriate promoters for use in such expression vectors include both constitutive promoters and inducible promoters, examples of which include a CMV promoter, and the metallothienein promoter.

Transfection is carried out as previously described and transfectants are selected for over-expression of PKR.

By over-expression of PKR is meant higher than normal levels of PKR activity. Such "normal" PKR activity or expression is reported as a range of PKR activity or expression, which is generally observed for a given type of cells which have not been transfected with a vector encoding PKR, are unstimulated (not induced or primed) and uninfected. It will be understood that the range of normal PKR activity for a given type of cell may vary somewhat dependent upon culture conditions.

Higher than normal PKR expression means at least 150%, preferably at least 200 or 300%, and more preferably 500% or more, of the normal PKR level. The PKR-overexpressing cell culture may be constitutive for PKR over-expression or inducible for PKR over-expression, depending on the particular method used to isolate or prepare the culture.

Preferably the PKR-overexpressing cell line will be inducible for PKR over-expression in order to regulate the level of PKR available for cytokine induction.

Similarly, preferably the cell culture will be inducible for overexpression of a protein which interferes with the apoptotic process or for the expression of a modified form of a protein which facilitates the apoptotic process, in order to regulate the apoptotic process in conjunction with PKR expression for optimal cytokine induction.

The activity of PKR and apoptosis-associated proteins may be determined by any of the methods known in the art. Exemplary assays for PKR expression include autophosphorylation assays, assay for eIF2α, Western blot, and (reverse transcriptase polymerase chain reaction) RT-PCR for PKR mRNA. Similarly, the expression of apoptosis-associated proteins may be determined by Western blot, and RT-PCR.

Any of a number of known cell types, modified in a manner to inhibit apoptosis, are useful for making a PKR-overexpressing cell line.

Any of a number of known cell cultures are useful as a parental strain for making a PKR-overproducing cell culture. Any cells normally capable of producing cytokines are suitable as the parental strain, as noted above. However, any cell line capable of producing a given cytokine or protein of interest may be employed in the methods of the invention. Human cell lines capable of cytokine or other protein production may be obtained by any number of methods that are well known in the art, including isolation of primary cell lines, or such cell lines may be obtained from commercial sources. In most cases, cells capable of producing a given cytokine or other protein are cultured in any suitable medium.

In some cases, additional steps are taken to enhance PKR expression by human cells, particularly, priming the PKR-expressing cells. Such priming may include treating with a priming agent, such as 1) G-CSF, EGF, TNF-alpha or TNF-beta, IL-1, interferons including IFN-alpha, IFN-beta, IFN-gamma or chemokines including IL-8, macrophage inflammatory proteins including MIP-1a & -1b and monocyte chemotactice proteins (MCP); 2) other cellular signaling factors such as phorbol myristate acetate (PMA), calcium ionophores, sodium butyrate or endotoxin; 3) poly IC, double-stranded RNA or viral analog; 4) cellular stress signals that can activate PKR including heat shock or pathogen infections including virus.

Such treating may include adding a microbial or non-microbial inducer to the cell culture. Preferably, the inducer will be a non-microbial inducer, *for example,* poly IC or poly rIC.

### VII. EVALUATION OF CYTOKINE OR OTHER PROTEIN EXPRESSION

In order to evaluate the expression of a cytokine or other protein of interest by a PKR-overexpressing cell line, which has been treated in manner effective to inhibit apoptosis, assays can be carried out at the protein level, the RNA level or by use of functional bioassays particular to the individual cytokine or other protein being expressed.

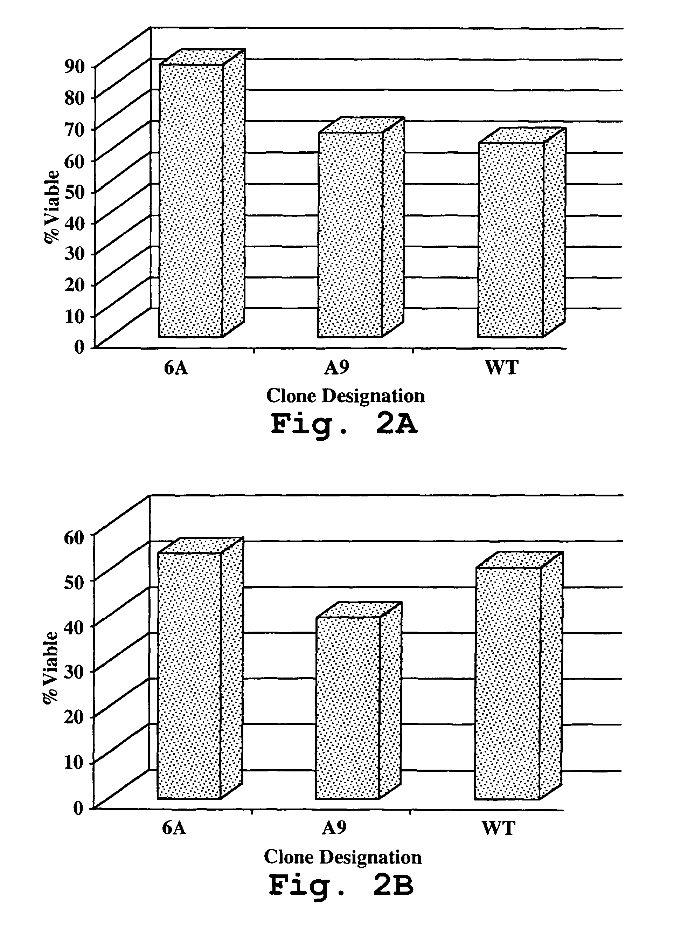

To demonstrate the invention, cells lines transfected with a PKR gene, and with both PKR and BclX_{L} genes were tested for cell viability under conditions of cytokine induction, with both polyIC and Sendai virus dsRNA, as detailed in Example 3. In these studies, "6A" cells were transfected with both PKR and Bcl-X_{L} genes; "A9" cells, with the PKR gene only; and "WT", non-transformed. The cells were tested under conditions of PKR overproduction (which would occur in the 6A and A9 cells), following cytokine induction with either polyIC or Sendai virus RNA. As seen from the data in Figs. 2A and 2B, inhibiting apoptosis in PKR overproducing cells significantly increased cell viability under conditions of cytokine induction, and even enhanced viability over WT cells (no PKR overproduction).

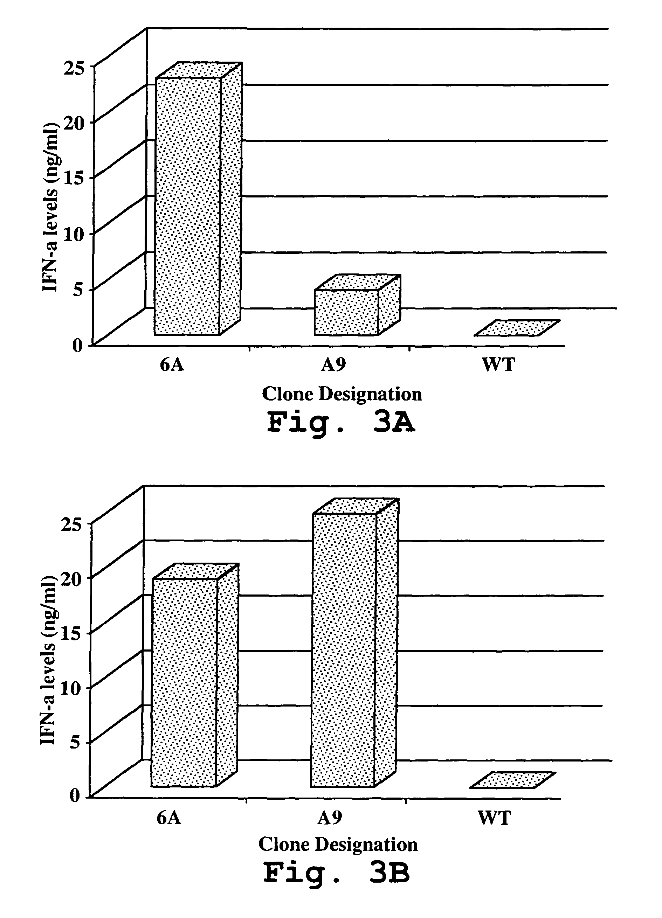

In a related experiment, also detailed in Example 3, expression levels of IFN-alpha were measured in the same three cells lines, again under conditions of PKR overproduction and cytokine induction with either Sendai virus or polyIC. From the data in Fig. 3A, it is seen that PKR overproduction (6A and A9 vs WT) significantly enhances cytokine production, and that a several fold further enhancement in cytokine production was observed by inhibiting apoptosis during cytokine-induction conditions (6A vs. A9). The analogous results in Fig. 3B also illustrate the significant enhancement in cytokine induction achieved with PKR overproduction (6A and A9 vs. WT). The higher levels of cytokine production observed in A9 vs. 6A cells may reflect a temporal effect and does not consider the overall amount of cytokine production during the period of cell viability.

Immunoassays for a particular cytokine or other proteins may be carried out using procedures routinely employed by those of skill in the art. Such immunoassays can be used to qualitatively and quantitatively analyze expression of a cytokine or other protein of interest.

In general, a purified form of the cytokine or other protein of interest, is either obtained from a natural source or produced recombinantly in transfected cells, and purified using standard techniques for protein purification. The purified protein is then used to produce either monoclonal or polyclonal antibodies specific to the expressed protein, and which can be used in various immunoassays. (See, *for example,* Harlow and Lane, 1988). Exemplary assays include ELISA, competitive immunoassays, radioimmunoassays, Western blots, indirect, immunofluorescent assays and the like.

In general, kits which are commercially available may be used for the quantitative immunoassay of the expression level of known cytokines or other proteins.

In addition, the functional expression of eukaryotic proteins is well known. Exemplary methods are described in Sambrook, *et al.,* 1989, expressly incorporated by reference herein. Briefly, cells are transfected with a suitable expression vector and cultured under conditions effective to result in expression of the cytokine or other protein of interest into the culture medium or on the surface of the transfected cell.

### Example 1

### Preparation of Plasmids pEF-FLAG-Bcl-X_{L} and pcDNA-FLAG-PKR

### 1. Preparation of pEF-FLAG-Bcl-X_{L}

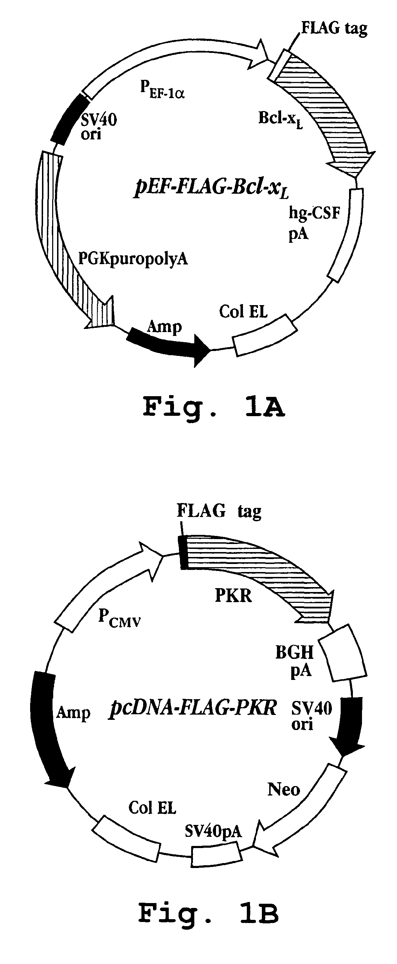

The pEF-FLAG-Bcl-X_{L} vector (Huang, et al., 1997) in Figure 1A contains a full length cDNA encoding the anti-apoptotic Bcl-X_{L} protein operably linked to the strong elongation factor 1 alpha (EF-1 alpha) promoter. An additional salient feature of the vector is the N-terminal FLAG epitope (Hopp et al., 1988) that was added to the Bcl-X_{L} protein to facilitate selection of cell lines that express high levels of Bcl-X_{L}.

The vector also includes i) a polyadenylation signal and transcription termination sequence to enhance mRNA stability; ii) a SV40 origin for episomal replication and simple vector rescue; iii) an ampicillin resistance gene and a ColE1 origin for selection and maintenance in E. coli; and iv) a puromycin resistance marker (Puro) to allow for selection and identification of the plasmid-containing eukaryotic cells after transfection of a Bcl-X_{L} and PKR.

### 2. Preparation of pcDNA-FLAG-PKR

The pcDNA-FLAG-PKR vector in Figure 1B contains cDNA encoding the full-length human PKR molecule (551 amino acids; Meurs, et al., 1990; GenBank Accession No. NM002759) modified by the polymerase chain reaction to include the N terminal FLAG tag (Hopp et al.,1988) encoding the sequence MDYKDDDDK, and inserted into the eukaryotic expression vector pcDNA3 (Invitrogen), such that the FLAG-PKR coding sequence was expressed under the control of the CMV promoter.

The vector, termed pcDNA-FLAG-PKR, contains various features suitable for PKR transcription, including: i) a promoter sequence from the immediate early gene of the human CMV for high level mRNA expression; ii) a polyadenylation signal and transcription termination sequence from the bovine growth hormone (BGH) gene to enhance mRNA stability; iii) a SV40 origin for episomal replication and simple vector rescue; iv) an ampicillin resistance gene and a ColE1 origin for selection and maintenance in E. coli; and v) a G418 resistance marker (Neo) to allow for selection and identification of the plasmid-containing eukaryotic cells after transfection.

A second PKR vector, designated pTRE-PKR, was prepared by inserting the same PKR cDNA into the gene-insertion site of a pTRE plasmid obtained from Clonetech. The pTRE plasmid is similar to the pFLAG used in making the first-described PKR vector, but contains a tetracycline-responsive element upstream of the CMV promoter used to control the inserted gene. In the studies reported in Example 3, the TRE function was not exploited, and so the operation of the two PKR vectors in transformed cells is expected to be essentially identical.

### Example 2

### Preparation of PKR Over-producing Namalwa Cell Lines 6A and A9

### 1. Preparation of Cell Line 6A

The human B lymphoblastoid cell line Namalwa (WT) was transfected sequentially with the plasmids, pEF-FLAG-Bcl-X_{L} and pcDNA-FLAG-PKR. The transfected cell line is termed 6A.

Stable transfectants were obtained by electroporation of 4x10⁶ exponentially growing Namalwa cells with 15ug of the pEF-FLAG-Bcl-X_{L} plasmid in DMEM/F12 (+10% FBS) using a Gene Pulser apparatus (BioRad) set at 800 uF, 300V. Bulk populations of stable transformants were obtained by selection with 2 ug/ml puromycin (Gibco-BRL) for 3-4 weeks and screened for Bel-X_{L} expression by flow cytometry as follows. The bulk transfectants were washed, permeabilized with acetone and subsequently stained with 2 ug/ml mouse anti-FLAG M2 monoclonal antibody (IBI) and then with phycoerythrin conjugated goat anti-mouse IgG (1ug/ml; Becton-Dickinson). Cells were analyzed in the FACScan, live and dead cells being discriminated on the basis of their forward and side light-scattering properties and Bcl-X_{L} expressing cells by their level of fluorescence intensity. High level Bcl-X_{L} expressing transformants (Namalwa-Bcl-X_{L}) were then transfected with pcDNA-FLAG-PKR.

Stable high level Bcl-X_{L} expressing transfectants were obtained by electroporation of 4x10⁶ exponentially growing Namalwa- Bel-X_{L} cells with 15 ug of the pcDNA-FLAG-PKR plasmid in DMEM/F12 (+10% FBS) using a Gene Pulser apparatus (BioRad) set at 800uF, 300V. Bulk populations of stable transformants were obtained by selection with 2 mg/ml geneticin (G418, Gibco-BRL) for 3-4 weeks. Clonal lines were subsequently obtained by limiting dilution cloning and analyzed for Bcl-X_{L} and PKR expression by Western blot analysis (Huang et al.,1997). The proteins were identified using 2 ug/ml anti-FLAG M2 antibody followed by goat anti-mouse IgG-peroxidase conjugate and ECL detection (Amersham).

### 2. Preparation of Cell Line A9

Stable high level PKR expressing transfectants were obtained by electroporation of 4x10⁶ exponentially growing Namalwa cells with 15 ug of the pTRE-PKR plasmid in DMEM/F12 (+10% FBS) using a Gene Pulser apparatus (BioRad) set at 800uF, 300V. Bulk populations of stable transformants were obtained by selection with 2 mg/ml geneticin (G418, Gibco-BRL) for 3-4 weeks. Clonal lines were subsequently obtained by limiting dilution cloning and analyzed for PKR expression by Western blot analysis (Huang et al.,1997).

### Example 3

### Characterization of a Bcl-X_{L} and PKR Over-producing Namalwa Cell Line

### 1. Increased Cell Viability

Wildtype Nowalwa cells (WT) and the A9 and 6A cells from Example 2 were examined for cell viability in culture under conditions of PKR overproduction and cytokine induction. Specifically, PKR and Bcl-X_{L} double-transfected Namalwa cells (the 6A cell line), PKR-transfected Namalwa cells (the A9 cell line) and parental Namalwa cells (WT) were cultured at 2.5x10⁵ cells/ml in DMEM/F12 medium supplemented with 10% FBS. The cells were treated with 20 mM PMA (primer) for 20 hr followed by treatment with either 200 ug/ml poly r(I):poly r(C) and 10 ug/ml DEAE Dextran (poly IC induction) for 72 hr. or 200 HAU/1x10⁶ cells of Sendai virus for 48 hr. Following treatment, cell viability was assessed by flow cytometry on a FACScan.

Figures 2A and 2B show that poly IC induction of all three cell lines resulted in significantly less cell viability than Sendai virus induction of the cells at the respective, indicated time periods. With poly IC induction, 54% of 6A, 40% of A9 and 51% of WT cells remained viable, whereas with Sendai virus induction, 87% of 6A, 66% of A9 and 63% of WT cells remained viable.

With both induction protocols, the 6A cell line, which overexpresses both the anti-apoptotic protein Bcl-X_{L} and PKR, showed greater viability than the A9 cell line which overexpresses PKR, but is not inhibited for apoptosis.

### 2. Increased Expression of Interferon-alpha

The level of IFN-alpha production was also analyzed in the three cell lines following cytokine induction by poly IC and Sendai virus, both under conditions of PKR overproduction. The culture supernatants were collected and analyzed for IFN-alpha levels by ELISA according to the procedure provided by the supplier of the ELISA kits (R&D Systems). The results are shown in Fig. 3A and 3B, and discussed above.

From the foregoing, it can be seen how various objects and features of the invention are met. Those skilled in the art can now appreciate from the foregoing description that the broad teachings of the present invention can be implemented in a variety of forms. Therefore, while this invention has been described in connection with particular embodiments and examples thereof, the true scope of the invention should not be so limited.

## Claims

1. A method for producing cytokines in a human cell culture, comprising:
(a) culturing a human cell line capable of producing cytokines and transfected with (i) a first vector containing DNA encoding a protein effective to inhibit cell apoptosis under the control of a first promoter; and (ii) a second vector containing DNA encoding double-stranded-RNA-dependent-kinase (PKR) under the control of a second promoter, under culture conditions in which PKR is overproduced in the transfected cells, as evidenced by levels of PKR in the transfected cell line which are higher than those obtained in the human cell line which is not transfected with the first and second vectors, when grown under the same culture conditions,
(b) treating the cultured, PKR overproducing human cell line with double-stranded RNA (dsRNA), and
(c) collecting one or more cytokines produced by the cultured, treated cell line.

2. The method of claim 1, wherein the cultured cell line is prepared by transfecting a human cell capable of producing cytokines successively with the first vector and the second vector.

3. The method of claim 1 or 2, wherein the protein effective to inhibit apoptosis is selected from the group consisting of B-cell Lymphoma/Leukemia-2 gene (Bcl-2a), B-cell Lymphoma/Leukemia- X_{L} (Bcl-X_{L}), a modified form of eukaryotic translation initiation factor 2 alpha (eIF-2 alpha), eukaryotic translation initiation factor (eIF-3), a modified form of Fas-associated death domain (FADD), a modified form of Bcl-X_{S}, a modified form of Bcl-2-homologous antagonist/killer (BAK) and a modified form of BAX.

4. The method of claim 3, wherein the protein effective to inhibit apoptosis is Bcl-2a or Bcl-X_{L}

5. The method of any one of claims 1 to 4, wherein the first or second promoter is an inducible promoter.

6. The method of claim 5, wherein the inducible promoter is a metallothionein promoter.

7. The method of any one of claims 1 to 6, wherein the cytokine(s) produced are selected from the group consisting of:
i) interferons selected from the group consisting of IFN-alpha and IFN-beta, IFN-gamma;
ii) tumor necrosis factors (TNF) selected from the group consisting of TNF-alpha, TNF-beta and TNF soluble receptors (sTNF-R);
iii) interleukins (IL) selected from the group consisting of IL-2, IL-3, IL-4, IL-5, IL-6, IL-7, IL-8, IL-11 and IL-12;
iv) colony stimulating factors selected from the group consisting of granulocyte colony stimulating factors (G-CSF) and granulocyte-macrophage colony stimulating factor (GM-CSF);
v) angiogenic factors selected from the group consisting of fibroblast growth factor (FGF), vascular endothelial growth factor (VEGF), platelet-derived growth factors 1 and 2 (PDGF 1 and 2);
vi) chemokines selected from the group consisting of Regulated Upon Activation Normally T-Expressed Secreted (RANTES), macrophage inflammatory proteins (MIP) including MIP-1alpha and MIP-2alpha and monocyte chemotactic protein 1 (MCP);
vii) anti-angiogenic factors selected from the group consisting of angiostatin and endostatin;
viii) leukemia inhibitory factor (LIF);
ix) ciliary neorotrophic factor and cardiotrophin; and
x) oncostatins, including oncostatin M.

8. The method of any one of claims 1 to 7, wherein the cultured human cell line is derived from a parental strain cell line selected from the group consisting of fibroblasts or immune cells, : B cells, T cells, monocytes, neutrophils, natural killer cells, pro-monocytic U937 cells, Namalwa cells, MRC-5 cells, WI-38 cells, Flow 1000 cells, Flow 4000 cells, FS-4, FS-7 cells, MG-63 cells, CCRF-SB cells, CCRF-CEM, Jurkat cells, WIL2 cells and THP-1 cells.

9. A method for producing cytokines in a human cell culture by culturing a human cytokine-producing cell under conditions of PKR overproduction and cytokine induction, **characterized in that** for increasing the viability of the cells, cells are employed as the cell line which have been transfected with a vector containing DNA encoding a protein effective to inhibit apoptosis in the cells.

10. The method of claim 9, wherein the cell line is also transfected with a vector containing DNA expressing PKR.

11. The method of claim 9 or 10, wherein the DNA encoding a protein effective to inhibit apoptosis in the cells encodes a protein selected from the group consisting of Bcl-2, Bcl-X_{L}, a modified form of eukaryotic translation initiation factor 2 alpha (eIF-2 alpha), eukaryotic translation initiation factor (eIF-3), a modified form of Fas-associated death domain (FADD), a modified form of Bcl-X_{S}, a modified form of BAK and a modified form of BAX, operably linked to a second promoter, under conditions effective to result in expression of the protein by the cells of the transfected cell line.

12. The method of claim 11, wherein the protein effective to inhibit apoptosis is Bcl-2a or Bcl-X_{L}

## Patentansprüche

1. Verfahren zur Herstellung von Cytokinen in einer menschlichen Zellkultur, umfassend
(a) Züchten einer menschlichen Zelllinie, welche in der Lage ist, Cytokine zu produzieren und transfiziert ist mit (i) einem ersten Vektor, welcher DNA enthält, die ein Protein codiert, das die Hemmung von Zellapoptose bewirkt, unter der Kontrolle eines ersten Promotors und (ii) einem zweiten Vektor, welcher DNA enthält, die doppelsträngige RNA-abhängige Kinase (PKR) codiert, unter der Kontrolle eines zweiten Promotors, unter Kulturbedingungen, bei welchen PKR in den transfizierten Zellen überproduziert wird, wie durch die PKR-Mengen in der transfizierten Zelllinie nachgewiesen werden kann, welche höher sind als die jene, welche in menschlichen Zelllinien erhalten werden, die nicht mit dem ersten und dem zweiten Vektor transfiziert sind, wenn sie unter denselben Kulturbedingungen angezogen werden,
(b) Behandlung der gezüchteten, PKR-überproduzierenden menschlichen Zelllinie mit doppelsträngiger RNA (dsRNA), und
(c) Gewinnung eines oder mehrerer Cytokine, welche von der gezüchteten, behandelten Zelllinie produziert werden.

2. Verfahren nach Anspruch 1, wobei die gezüchtete Zelllinie durch die nacheinanderfolgende Transfizierung einer menschlichen Zelle, welche in der Lage ist, Cytokine zu produzieren, mit dem ersten und dem zweiten Vektor erhalten wird.

3. Verfahren nach Anspruch 1 oder 2, wobei das Protein, welches die Hemmung von Apoptose bewirkt, ausgewählt ist aus der Gruppe bestehend aus B-Zelllymphom/Leukämie-2-Gen (Bcl-2a), B-Zelllymphom/Leukämie-X_{L} (Bcl-X_{L}), einer modifizierten Form des eukaryontischen Translationsinitiationsfaktors 2 Alpha (eIF-2 Alpha), dem eukaryontischen Translationsinitiationsfaktor (eIF-3), einer modifizierten Form der Fas-assoziierten Todesdomäne (FADD), einer modifizierten Form von Bcl-X_{S}, einer modifizierten Form des Bcl-2- homolgen Antagonisten/Killers (BAK) und einer modifizierten Form von BAX.

4. Verfahren nach Anspruch 3, wobei das Protein, welches die Hemmung von Apoptose bewirkt, Bcl-2a oder Bel-X_{L} ist.

5. Verfahren nach einem der Ansprüche 1 bis 4, wobei der erste oder der zweite Promotor ein induzierbarer Promotor ist.

6. Verfahren nach Anspruch 5, wobei der induzierbare Promotor ein Metallothionein-Promotor ist.

7. Verfahren nach einem der Ansprüche 1 bis 6, wobei das/die produzierte(n) Cytokin(e) ausgewählt ist/sind aus der Gruppe bestehend aus:
(i) Interferonen, ausgewählt aus der Gruppe bestehend aus IFN-α, IFN-β und IFN-γ;
(ii) Tumornekrosefaktoren (TNF), ausgewählt aus der Gruppe bestehend aus TNF-α, TNF-β und löslichen TNF-Rezeptoren (sTNF-R);
(iii) Interleukinen (IL), ausgewählt aus der Gruppe bestehend aus IL-2, IL-3, IL-4, IL-5, IL-6, IL-7, IL-8, IL-11 und IL-12;
(iv) koloniestimulierenden Faktoren, ausgewählt aus der Gruppe bestehend aus Granulocytenkolonie-stimulierenden Faktoren (G-CSF) und Granulocyten-Makrophagen-Kolonie-stimulierendem Faktor (GM-CSF);
(v) angiogenen Faktoren, ausgewählt aus der Gruppe bestehend aus Fibroblastenwachstumsfaktor (FGF), vaskulären endothelialem Wachstumsfaktor (VEGF), plättchenabgeleitetem Wachstumsfaktor 1 und 2 (PDGF 1 und 2);
(vi) Chemokinen, ausgewählt aus der Gruppe bestehend aus Regulated Upon Activation Normally T-Expressed Secreted (RANTES), Makrophagen-Entzündungsproteinen (MIP) einschließlich MIP-1α und MIP-2α und Monocyten-chemotaktischem Protein 1 (MCP);
(vii) anti-angiogenen Faktoren, ausgewählt aus der Gruppe bestehend aus Angiostatin und Endostatin;
(viii) Leukämie-inhibierendem Faktor (LIF);
(ix) ziliärem neurotrophem Faktor und Cardiotrophin; und
(x) Oncostatinen, einschließlich Oncostatin M.

8. Verfahren nach einem der Ansprüche 1 bis 7, wobei die gezüchtete menschliche Zelllinie abgeleitet ist aus einer elterlichen Stammzelllinie ausgewählt aus der Gruppe bestehend aus Fibroblasten oder Immunzellen, B-Zellen, T-Zellen, Monocyten, Neutrophilen, natürlichen Killerzellen, pro-monocytischen U937-Zellen, Namalwa-Zellen, MRC-5-Zellen, WI-38-Zellen, Flow 1000-Zellen, Flow 4000-Zellen, FS-4, FS7-Zellen, MG-63-Zellen, CCRF-SB-Zellen, CCRF-CEM, Jurkat-Zellen, WIL2-Zellen und THP-1-Zellen.

9. Verfahren zur Herstellung von Cytokinen in einer menschlichen Zellkultur durch das Züchten einer menschlichen Cytokin-produzierenden Zelle unter Bedingungen der PKR-Überproduktion und Cytokininduktion, **dadurch gekennzeichnet, dass** zur Steigerung der Lebensfähigkeit der Zellen Zellen als Zelllinien verwendet werden, welche mit einem Vektor transfiziert wurden, der DNA enthält, welche ein Protein codiert, das die Hemmung von Apoptose in den Zellen bewirkt.

10. Verfahren nach Anspruch 9, wobei die Zelllinie ebenfalls mit einem Vektor transfiziert ist, welcher PKR-exprimierende DNA enthält.

11. Verfahren nach Anspruch 9 oder 10, wobei die DNA, welche ein Protein codiert, das die Hemmung von Apoptose in den Zellen bewirkt, ein Protein codiert, das ausgewählt ist aus der Gruppe bestehend aus Bcl-2, Bcl-X_{L}, einer modifizierten Form des eukaryontischen Translationsinitiationsfaktors 2 Alpha (eIF-2 Alpha), dem eukaryontischen Translationsinitiationsfaktor (eIF-3), einer modifizierten Form der Fas-assoziierten Todesdomäne (FADD), einer modifizierten Form von Bel-X_{S}, einer modifizierten Form von BAK und einer modifizierten Form von BAX, welche funktionell mit einem zweiten Promotor verknüpft sind, unter Bedingungen, die die Expression des Proteins durch die Zellen der transfizierten Zelllinie bewirken.

12. Verfahren nach Anspruch 11, wobei das Protein, welches die Hemmung von Apoptose bewirkt, Bcl-2a oder Bcl-X_{L} ist.

## Revendications

1. Méthode pour produire des cytokines dans une culture cellulaire humaine, comprenant :
(a) la culture d'une lignée cellulaire humaine capable de produire des cytokines et transfectée avec (i) un premier vecteur contenant de l'ADN codant une protéine efficace pour inhiber l'apoptose cellulaire sous le contrôle d'un premier promoteur ; et (ii) un second vecteur contenant de l'ADN codant une kinase dépendante de l'ARN double brin (PKR) sous le contrôle d'un second promoteur, dans des conditions de culture dans lesquelles PKR est surproduite dans les cellules transfectées, tel que montré par les niveaux de PKR dans la lignée cellulaire transfectée qui sont supérieurs à ceux obtenus dans la lignée cellulaire humaine qui n'est pas transfectée avec les premier et second vecteurs, lorsqu'elles sont cultivées dans les mêmes conditions,
(b) le traitement de la lignée cellulaire humaine cultivée surproduisant PKR avec de l'ARN double brin (ARNdb), et
(c) la récolte d'une ou plusieurs cytokines produites par la lignée cellulaire traitée cultivée.

2. Méthode selon la revendication 1, dans laquelle la lignée cellulaire cultivée est préparée par transfection d'une cellule humaine capable de produire des cytokines avec successivement le premier vecteur et le second vecteur.

3. Méthode selon la revendication 1 ou 2, dans laquelle la protéine efficace pour inhiber l'apoptose est choisie dans le groupe constitué du gène Lymphome de cellule B /Leucémie-2 (Bcl-2a), de Lymphome de cellule B /Leucémie-X_{L} (Bcl-X_{L}) ; d'une forme modifiée du facteur d'initiation de la traduction eucaryote alpha 2 (eIF-2 alpha), du facteur d'initiation de la traduction eucaryote (eIF-3), d'une forme modifiée du domaine de mort associé à Fas (FADD), d'une forme modifiée de Bcl-X_{S}, d'une forme modifiée de l'antagoniste homologue de Bcl-2/tueur (BAK) et d'une forme modifiée de BAX.

4. Méthode selon la revendication 3, dans laquelle la protéine efficace pour inhiber l'apoptose est Bcl-2a ou Bel-X_{L}.

5. Méthode selon l'une quelconque des revendications 1 à 4, dans laquelle le premier ou le second promoteur est un promoteur inductible.

6. Méthode selon la revendication 5, dans laquelle le promoteur inductible est un promoteur de métallothionéine.

7. Méthode selon l'une quelconque des revendications 1 à 6, dans laquelle la(les) cytokine(s) produites sont choisies dans le groupe constitué de :
i) interférons choisis dans le groupe constitué de IFN-alpha et IFN-bêta, IFN-gamma ;
ii) facteurs de nécrose tumorale (TNF) choisis dans le groupe constitué de TNF-alpha, TNF-bêta et récepteurs solubles de TNF (sTNF-R) ;
iii) interleukines (IL) choisies dans le groupe constitué de IL-2, IL-3, IL-4, IL-5, IL-6, IL-7, IL-8, IL-11 et IL-12 ;
iv) facteurs de stimulation de colonies choisis dans le groupe constitué de facteurs de stimulation de colonies granulocyte (G-CSF) et facteur de stimulation de colonies granulocyte-macrophage (GM-CSF) ;
v) facteurs angiogéniques choisis dans le groupe constitué du facteur de croissance fibroblastique (FGF), facteur de croissance de l'endothélium vasculaire (VEGF), facteur de croissance dérivé des plaquettes 1 et 2 (PDGF 1 et 2) ;
vi) chimiokines choisies dans le groupe constitué de RANTES (Regulated Upon Activation Normally T-Expressed Secreted), protéines inflammatoire des macrophages (MIP) incluant MIP-1 alpha et MIP-2 alpha et la protéine chimiotactic monocytaire 1 (MCP) ;
vii) facteurs antiangiogéniques choisis dans le groupe constitué de l'angiostatine et endostatine ;
viii) facteur inhibiteur de leucémie (LIF);
ix) facteur neurotrophique ciliaire et cardiotrophine ; et
x) oncostatines, incluant l'oncostatine M.

8. Méthode selon l'une quelconque des revendications 1 à 7, dans laquelle la lignée cellulaire humaine cultivée est dérivée d'une lignée cellulaire souche parentale choisie dans le groupe constitué de fibroblastes ou cellules immunitaires, lymphocytes B, lymphocytes T, monocytes, neutrophiles, cellules tueuses naturelles, cellules U937 pro-monocytiques, cellules de Namalwa, cellules MRC-5, cellules WI-38, cellules Flow 1000, cellules Flow 4000, FS-4, cellules FS-7, cellules MG-63, cellules CCRF-SB, CCRF-CEM, cellules Jurkat, cellules WIL2 et cellules THP-1.

9. Méthode pour produire des cytokines dans une culture cellulaire humaine par culture d'une cellule productrice de cytokines humaine dans des conditions de surexpression de PKR et induction de cytokine, **caractérisée en ce que**, pour augmenter la viabilité des cellules, des cellules sont utilisées comme la lignée cellulaire qui a été transfectée avec un vecteur contenant de l'ADN codant une protéine efficace pour inhiber l'apoptose dans les cellules.

10. Méthode selon la revendication 9, dans laquelle la lignée cellulaire est également transfectée avec un vecteur contenant de l'ADN exprimant PKR.

11. Méthode selon la revendication 9 ou 10, dans laquelle l'ADN codant une protéine efficace pour inhiber l'apoptose dans les cellules code une protéine choisie dans le groupe constitué de Bcl-2, Bel-X_{L}, une forme modifiée du facteur d'initiation de la traduction eucaryote alpha 2 (eIF-2 alpha), du facteur d'initiation de la traduction eucaryote (eIF-3), d'une forme modifiée du domaine de mort associé à Fas (FADD), d'une forme modifiée de Bcl-X_{S}, d'une forme modifiée de BAK et d'une forme modifiée de BAX, lié de manière opérationnelle à un second promoteur, dans des conditions efficaces pour obtenir une expression de la protéine par les cellules de la lignée cellulaire transfectée.

12. Méthode selon la revendication 11, dans laquelle la protéine efficace pour inhiber l'apoptose est Bcl-2a ou Bel-X_{L}.
